# FASCICULE DE BREVET EUROPEEN

(11) **EP 3 082 813 B1**
(45) Date de publication et mention de la délivrance du brevet: **16.05.2018**
(21) Numéro de dépôt: 14830833.1
(22) Date de dépôt: 18.12.2014
(51) Int. Cl.: A61K 9/00, A61K 31/454, A61P 17/00

(54) **UTILISATION DU NARATRIPTAN DANS LE TRAITEMENT DE LA ROSACEE**
VERWENDUNG VON NARATRIPTAN ZUR BEHANDLUNG VON ROSACEA
USE OF NARATRIPTAN IN THE TREATMENT OF ROSACEA

(30) Priorité: 19.12.2013 FR 1362978
(43) Date de publication de la demande: 26.10.2016
(73) Titulaire: Galderma Research & Development, 06410 Biot (FR)
(72) Inventeur: RIVIER, Michel, F-06100 Nice (FR); CARLAVAN, Isabelle, 06130 Grasse (FR); AUBERT, Jérôme, 06130 Grasse (FR)
(74) Mandataire: Cabinet Becker et Associés
(86) Numéro de dépôt international: PCT/FR2014/053440
(87) Numéro de publication internationale: WO 2015/092309

(56) Documents cités:
- WO-A1-2011/048496
- FR-A1- 2 758 263
- JULIA SPOENDLIN ET AL: "Migraine, triptans, and the risk of developing rosacea", JOURNAL OF THE AMERICAN ACADEMY OF DERMATOLOGY, vol. 69, no. 3, 1 septembre 2013 (2013-09-01), pages 399-406, XP055123144, ISSN: 0190-9622, DOI: 10.1016/j.jaad.2013.03.027
- BALDWIN H E: "Diagnosis and treatment of rosacea: State of the art", JOURNAL OF DRUGS IN DERMATOLOGY, STRATEGIC COMMUNICATION IN DERMATOLOGY, NEW YORK, NY, US, vol. 11, no. 6, 1 juin 2012 (2012-06-01), pages 725-730, XP009172413, ISSN: 1545-9616

## Description

L'invention se rapporte à une composition pharmaceutique topique, notamment dermatologique, pour son utilisation pour la prévention ou le traitement de la rosacée et notamment la rosacée érythématotélangiectasique.

De nombreux troubles cutanés inflammatoires entraînent souvent des éruptions cutanées inesthétiques et douloureuses, de l'acné, des télangiectasies, et des éruptions cutanées semblables à l'acné, tels que des macules, nodules et pustules qui peuvent suinter ou croûter.

Par exemple, la rosacée est une dermatose inflammatoire chronique affectant principalement la partie médiane du visage et les paupières de certains adultes. Elle est caractérisée par un érythème télangiectasique, une sécheresse de la peau, des papules et des pustules.

Classiquement, la rosacée se développe chez les adultes entre l'âge de 30 à 50 ans; elle atteint plus fréquemment les femmes bien que l'affection soit généralement plus sévère chez les hommes. La rosacée est chronique et persiste des années avec des périodes d'exacerbation et de rémission.

La rosacée, anciennement connue sous le nom « d'acné rosacée », n'est pas une affection du follicule pilosébacé comme l'acné juvénile mais une affection primitivement vasculaire dont le stade inflammatoire est dépourvu de kystes et de comédons caractéristiques de l'acné vulgaire.

L'étiologie de la rosacée est encore mal comprise, bien que de nombreuses théories aient été élaborées. La thèse la plus commune est basée sur la présence caractéristique du parasite *Demodex folliculorum* chez les patients atteints de rosacée. D'autres facteurs ont été décrits comme pouvant contribuer au développement de la rosacée, tels que les facteurs psychologiques, environnementaux (exposition au soleil, température, humidité), immunologiques, émotionnels (stress), alimentaires (alcool, épices), hormonaux, vasculaires, des troubles gastro-intestinaux, voire une infection par *Helicobacter pilori*.

La rosacée peut être classée de la manière suivante :
- Type I: Rosacée érythématotélangiectasique, se caractérisant principalement par un érythème centrofacial persistant et des rougissements épisodiques ou bouffées de chaleur (« flush »). Souvent, ce type se caractérise également par un oedème, des plaques rugueuses et l'apparition de vaisseaux sanguins dilatés (télangectasies) de même que par des sensations de brûlure et de piqûre.
- Type II: Rosacée papulopustulaire, se caractérisant par un érythème centrofacial persistant ainsi que par l'apparition de papules ou pustules centrofaciales transitoires, ressemblant à ceux de l'acné. Ces symptômes sont parfois accompagnés de sensations de brûlure et de piqûre. Ce type suit habituellement le type I ou se combine à ce dernier.
- Type III: Rosacée phymateuse marquée par l'épaississement de la peau et l'apparition de nodules irréguliers. Bien que le nez soit souvent la région la plus touchée, devenant très gros et couvert de boursouflures («rhinophyma»), d'autres localisations sont également observées : menton, front, joues et oreilles Ce type suit habituellement le type I et II ou se combine à ceux-ci.
- Type IV: Rosacée oculaire. Dans ce type de rosacée, les yeux rouges et irrités peuvent larmoyer et sembler injectés de sang. Les symptômes peuvent comprendre la sensation d'avoir un corps étranger dans l'oeil, un larmoiement excessif, une sensibilité à la lumière, une vision floue, une sensation de brûlure, de sécheresse ou de piqûre, un prurit et une alacrymie. Ils peuvent se produire avec ou sans la rosacée. La survenue peut intervenir avant, pendant ou après l'apparition des signes cutanés.

Les traitements actuels, qui sont dirigés sur le contrôle des éruptions et des rougeurs, l'inflammation de la peau, sont d'une efficacité limitée chez de nombreux patients et, en général, ne peuvent être utilisés que pour une durée limitée. Les traitements standards excluent les éléments potentialisateurs des pathologies tels que l'exposition au soleil, l'exposition au vent, la consommation d'alcool, les aliments épicés, irritants, les lotions nettoyantes, et les cosmétiques.

Classiquement, la rosacée est traitée oralement ou topiquement par des antibiotiques tels que les tétracyclines, l'érythromycine, la clindamycine, mais aussi par la vitamine A, l'acide salicylique, des agents antifongiques, des stéroïdes, le métronidazole (un agent antibactérien) ou par l'isotrétinoïne dans les formes sévères ou encore par des anti-infectieux tel que le peroxyde de benzoyle ou par l'acide azélaïque. On connaît également le traitement de la rosacée avec de l'ivermectine qui cible le parasite *Demodex folliculorum* présent sur la peau des patients (US 5,952,372).

Est également connu le traitement de la rosacée par des agonistes des récepteurs adrénergiques alpha-1 ou alpha-2 (US 2006/0171974, US 2005/0165079, US 2005/0020600). La brimonidine est un agoniste des récepteurs adrénergiques alpha-2 sélectif. La brimonidine s'est également avérée être utile dans le traitement de la rosacée et notamment de l'érythème causé par la rosacée, voir par exemple la demande de brevet US 2004/242588 de DeJovin et al.; la demande US 2005/020600 de Scherer; la demande US 2009/061020 de Theobald et al., ou des télangiectasies éventuellement causées par la rosacée, voir, par exemple, la demande de brevet US 2006/0264515.

Malheureusement, l'utilisation de ces médicaments comme par exemple, les antibiotiques, peut causer souvent des effets secondaires et peut engendrer des problèmes d'intolérance chez de nombreux patients. De plus, aucun des traitements existants ne permet de traiter et/ou prévenir efficacement l'ensemble des symptômes associés à la rosacée de manière durable.

WO 2011/048496 décrit l'utilisation des triptans en tant qu'inhibiteurs de la 5-hydroxytryptamine pour le traitement du psoriasis. FR 2 758 263 décrit l'utilisation des triptans en combinaison avec un antagoniste de la sérotonine de type 5HT2 pour traiter les peaux sensibles.

Tenant compte de ce qui précède, il existe donc un besoin d'un traitement plus efficace de la rosacée ayant une efficacité prolongée dans le temps, et qui présente moins d'effets secondaires, en particulier une composition conférant une plus grande tolérance du principe actif, tout en diminuant ses effets secondaires.

La Demanderesse propose de fournir ainsi une composition pharmaceutique topique pour le traitement de la rosacée plus efficace et prolongé, et notamment de la rosacée érythématotélangiectasique (de type I), avec potentiellement moins d'effets secondaires quelle que soit la durée d'application. En particulier, le traitement peut être efficace plus longtemps en ayant un effet rémanent à l'arrêt du traitement sur plusieurs semaines voire plusieurs mois. Cette composition pharmaceutique peut permettre en outre de d'éviter un effet rebond parfois observé en fin de traitement.

L'invention a pour objet les revendications 1 à 6.

L'invention a pour objet une composition pharmaceutique topique, comprenant du naratriptan, ou un sel pharmaceutiquement acceptable de celui-ci, dans un véhicule pharmaceutiquement acceptable pour le traitement et/ou la prévention de la rosacée, et plus préférentiellement de la rosacée érythématotélangiectasique, (dite de type I).

Préférentiellement, la composition selon l'invention est pour le traitement de la rosacée.

La présente divulgation décrit l'utilisation du naratriptan, ou d'un sel pharmaceutiquement acceptable de celui-ci, pour la préparation d'un médicament administré par voie topique destiné à la prévention et/ou au traitement de la rosacée, et plus préférentiellement de la rosacée érythématotélangiectasique, dite de type I.

La présente divulgation décrit également une méthode pour le traitement et/ou la prévention de la rosacée, et plus particulièrement encore pour le traitement et/ou à la prévention de la rosacée de type I, ladite méthode comprenant l'administration par voie topique d'une composition pharmaceutique topique comprenant une quantité thérapeutiquement efficace de naratriptan ou d'un sel pharmaceutiquement acceptable de celui-ci.

L'invention concerne encore du naratriptan ou d'un sel pharmaceutiquement acceptable de celui-ci administrée par voie topique pour une utilisation dans le traitement et/ou la prévention de la rosacée, de préférence encore de la rosacée de type I.

La présente divulgation décrit encore l'utilisation du naratriptan ou d'un sel pharmaceutiquement acceptable de celui-ci, pour la préparation d'une composition pharmaceutique topique, et notamment dermatologique, destinée à la prévention et/ou au traitement de la rosacée, et en particulier de la rosacée de type I.

En effet, la Demanderesse a découvert de manière surprenante que le naratriptan ou un sel pharmaceutiquement acceptable de celui-ci avait des propriétés combinées en terme d'activité, de sécurité, et de pénétration cutanée qui en faisait un candidat unique pour un traitement de la rosacée par voie topique . En particulier, le naratriptan présente une activité vasoconstrictrice sur les vaisseaux humains supérieure aux autres membres de la famille des triptans ainsi qu'une très bonne activité anti-inflammatoire neurogène, lui permettant d'être efficace dans la prévention et/ou le traitement de la rosacée et plus particulièrement de la rosacée érythémotélangiectasique (ou de type I). L'activité du naratriptan a été démontrée sur des vaisseaux issus du compartiment sous-cutané humain. Chez l'homme, chaque organe présente un tissu vasculaire particulier (artères ou veines) en termes d'organisation, de structure, de tonus et de réactivité vasculaire. Ces propriétés sont dues principalement à l'équipement en récepteurs. Par exemple, seuls les vaisseaux de petit diamètre de la peau expriment les récepteurs adrénergiques alpha 2, alors que les vaisseaux de petit, moyen et gros diamètre expriment les récepteurs adrénergiques alpha 1. Il s'ensuit des réponses contractiles différentes en fonction des activateurs ou inhibiteurs sélectifs pour les récepteurs adrénergiques alpha 1 ou alpha 2. Il est par conséquent impossible a priori, sans connaitre l'expression et la localisation d'un type de récepteur particulier, de prévoir le comportement d'un vaisseau sanguin de la peau en ce qui concerne sa contraction ou sa dilatation. Ce raisonnement s'applique également aux récepteurs pour la sérotonine 5HT1B et D, qui sont en particulier, activés par les triptans. De manière surprenante, la Demanderesse a découvert d'une part que les récepteurs aux triptans 5HT1B étaient exprimés sur les petits vaisseaux sanguins d'un diamètre allant de 200 à 500 µm (et les récepteurs aux triptans 5HT1D étaient présents sur les extrémités des nerfs sensoriels) mais également que ces vaisseaux étaient plus réactifs au naratriptan parmi l'ensemble des triptans testés. Cette découverte est d'autant moins évidente, que les paramètres physicochimiques des différents composés de la famille des triptans ne sont pas prédictifs d'une activité sur la peau de patients rosacée.

Au-delà de cet effet vasoconstricteur pur du naratriptan, une inhibition du relargage des neuropeptides vasodilatateurs peut amener un bénéfice supplémentaire sur le long terme en diminuant de manière significative l'inflammation neurogène.

L'inflammation neurogénique est une composante majeure de la rosacée de type I (érythémato-télangiectasique). Ce processus, dont l'inflammation est déclenchée par le système nerveux sensoriel, est caractérisé par des symptômes tels que rougeur, gonflement, et chaleur. Différents stimuli (température, boissons chaudes ou épicées...) sont responsables des « flushs » et peuvent conduire à un érythème facial permanent. Au niveau de la face, ces stimuli activent le système nerveux sensoriel trigéminé libérant localement des neuropeptides (CGRP, SP, PACAP...). Ces neuropeptides induisent alors une vasodilatation, responsable de la rougeur.

Ainsi, le naratriptan, compte tenu de son activité sur les récepteurs 5HT1D, est capable d'activer les récepteurs présents sur les extrémités des nerfs sensoriels de la peau et de réduire l'inflammation neurogène dans la peau.

Il agit en conséquence sur le flush et/ou l'érythème de la rosacée, en particulier de type I ou II. L'effet du naratriptan est très avantageux pour obtenir une efficacité prolongée dans le traitement de la rosacée, en particulier de type I ou II.

Dans un mode de réalisation préféré, la composition selon l'invention est utilisée pour le traitement de la rosacée érythématotélangiectasique, ou dite de type I.

Dans un mode de réalisation particulier, la composition selon l'invention est utilisée pour le traitement de la rosacée papulopustulaire, ou dite de type II.

Le naratriptan ou N-methyl-2-[3-(1-methylpiperidin-4-yl)-1H-indol-5-yl]ethanesulfonamide fait partie de la famille des triptans. Cette famille d'actifs est utilisée dans des médicaments destinés aux traitements aigus des crises de migraine. Ils sont également utilisés dans le traitement des algies vasculaires de la face. Il existe plusieurs types de triptans parmi lesquels le sumatriptan, premier triptan mis sur le marché dans les années 1990, le naratriptan, le zolmitriptan, l'élétriptan, l'almotriptan, le frovatriptan et le rizatriptan. Ils se présentent sous des formes galéniques diverses: injectable ou auto-injectables, formes orales ou spray nasal.

La demanderesse a découvert que, de manière surprenante, le naratriptan était le meilleur candidat de la famille des triptans pour une application par voie topique pour le traitement de la rosacée, sur la base de son efficacité, sa sécurité, et sa pénétration cutanée. En effet, le frovatriptan, le zolmitriptan et l'eletriptan sont suspectés d'être génotoxiques et carcinogènes, et le sumatriptan engendre des atteintes oculaires, notamment des opacités de la cornée. La rosacée est considérée comme une maladie dermatologique bénigne, bien que ses formes les plus avancées et sévères puissent présenter une morbidité importante pour les patients. Néanmoins, le rapport bénéfice/risque pour les patients traités doit être extrêmement élevé. En conséquence, ces derniers composés (frovatriptan, zolmitriptan, l'eletriptan, sumatriptan) présentent un potentiel de toxicité systémique après administration topique qui en font de très mauvais candidats pour un produit topique dans la rosacée. Le rizatriptan quant à lui, en raison de ses paramètres pharmacocinétiques, engendre des problèmes d'interactions médicamenteuses potentiels et génère un métabolite actif qui représente 14% de la molécule parente entrainant une complication de son développement.

Seuls le naratriptan et l'almotriptan présentent un profil de sécurité satisfaisant.

D'une manière générale, au-delà de l'activité et de l'absence de toxicité des composés pour une utilisation dans la rosacée par voie topique, une autre propriété est indispensable pour garantir que le composé puisse être efficace : il s'agit de la pénétration de ce composé dans la peau au travers de ses différentes couches pour atteindre les cibles pharmacologiques. En ce qui concerne la rosacée, ces cibles (les récepteurs 5HT-1B et D) sont situées d'une part sur les vaisseaux du derme et d'autre part sur les terminaisons nerveuses du derme et de l'épiderme. Cette propriété de pénétration est critique et incontournable pour un composé utilisé par voie topique. Elle se combine avec les propriétés d'activité intrinsèque du composé afin de garantir son efficacité clinique dans le traitement d'une affection cutanée. Dans un cas de pénétration faible ou nulle, le composé sera totalement inefficace cliniquement dans le traitement de la pathologie par la voie topique.

La demanderesse a découvert que, de manière surprenante, le naratriptan présentait une bien meilleure pénétration cutanée que l'almotriptan dans de la peau humaine. En particulier, la quantité totale de naratriptan retrouvée dans la peau humaine après application topique d'une formule concentrée à 2% dans une solution de tampon phosphate (PBS) est presque le double de celle retrouvée avec l'almotriptan utilisé dans le PBS à la même concentration (3,46% versus 1,86%). Cette propriété favorable pour le naratriptan est particulièrement inattendue. D'autant qu'il est impossible de prédire quel composé aura la pénétration la plus favorable sur la simple base de sa structure chimique et/ou de son profil physicochimique.

L'efficacité sur les vaisseaux sanguins de petit diamètre de la peau et la pénétration cutanée du naratriptan, plus importantes que celles des autres triptans, ainsi que son bon profil de sécurité, donnent à ce composé un profil inégalé pour le traitement de la rosacée par voie topique.

L'expression "sel(s) pharmaceutiquement acceptable(s) ", dans le présent contexte, désigne les sels d'un composé d'intérêt, de préférence pour une utilisation topique chez des mammifères, et qui possèdent l'activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine. Pour une revue sur les sels pharmaceutiquement acceptables, voir Berge et al. (J Pharm Sci. 1977 Jan;66(1):1-19).

Dans un mode de réalisation préférentiel, le sel de naratriptan est un chlorhydrate de naratriptan.

On entend par application topique, le fait d'appliquer ou d'étaler la composition selon l'invention à la surface de la peau ou d'une muqueuse.

Les compositions de l'invention comprennent en outre un véhicule pharmaceutiquement ou cosmétiquement acceptable, c'est-à-dire un véhicule adapté pour une utilisation topique en contact avec des cellules humaines, sans toxicité, intolérance, irritation, réponse allergique indue et similaire, et proportionné à un rapport avantage/risque raisonnable.

Dans la suite du texte, on utilisera le terme naratriptan pour définir indifféremment le naratriptan ou l'un de ses sels.

Les compositions de l'invention peuvent comprendre en outre tout additif ou adjuvant usuellement utilisé dans le domaine pharmaceutique, et plus particulièrement dermatologique, compatible avec le naratriptan.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Les compositions de la présente invention peuvent se présenter sous toutes les formes galéniques normalement utilisées pour une application topique, notamment sous forme de solutions, de lotions, de gels, d'onguents, d'émulsions de consistance liquide ou semi-liquide du type lait, obtenues par dispersion d'une phase grasse dans une phase aqueuse (H/E) ou inversement (E/H), ou de poudres, de tampons imbibes, de sprays, de suspensions ou émulsions de consistance molle, semi-liquide ou solide du type crème, pommade ou encore de Ces compositions sont préparées selon les méthodes usuelles.

De manière avantageuse, la composition comprend une pommade, une crème, une lotion ou un gel.

Dans un mode de réalisation, le terme " traitement " ou " traiter " désigne une amélioration, la prophylaxie, d'une maladie ou d'un trouble, ou au moins d'un symptôme pouvant être discerné de celui-ci.

Dans un autre mode de réalisation, " traitement " ou " traiter " désigne une amélioration, la prophylaxie, d'au moins un paramètre physique mesurable associé à la maladie ou au trouble étant traité, qui n'est pas nécessairement discernable chez ou par le sujet traité.

Dans un autre mode de réalisation supplémentaire, "traitement" ou "traiter" désigne l'inhibition ou le ralentissement de la progression d'une maladie ou un trouble, physiquement, par exemple, la stabilisation d'un symptôme discernable, physiologiquement, par exemple, la stabilisation d'un paramètre physique, ou les deux.

Dans un autre mode de réalisation, " traitement " ou " traiter " désigne le retard de l'apparition d'une maladie ou trouble.

Dans certains modes de réalisation, le composé d'intérêt est administré en tant que mesure préventive. Dans le présent contexte, "prévention" ou " prévenir " désigne une réduction du risque d'acquisition d'une maladie ou un trouble spécifié.

Au sens de la présente invention, par «patient» on entend tout mammifère, et plus particulièrement les êtres humains, hommes ou femmes.

La quantité réellement administrée de naratriptan dépend de l'effet thérapeutique recherché, et peut donc varier dans une large mesure. Ainsi, et selon une forme de réalisation préférée, la composition pharmaceutique est administrées 1 à 2 fois/jour. De préférence, le traitement peut avoir une durée allant de 1 semaine à 6 mois, renouvelable, et de préférence de 2 semaines à 4 mois.

Les cures peuvent être renouvelées en cycle avec ou sans période de repos.

Dans les compositions selon l'invention, le naratriptan est présent dans la composition à une concentration comprise entre 0,0001 % et 5 % en poids, par rapport au poids total de la composition le comprenant, de préférence comprise entre 0,001% et 3 % en poids par rapport au poids total de la composition.

Dans l'ensemble du présent texte, à moins qu'il ne soit spécifié autrement, il est entendu que lorsque des intervalles de concentrations sont donnés, ils incluent les bornes supérieures et inférieures dudit intervalle.

D'autres aspects et avantages de l'invention apparaîtront à la lecture des exemples qui suivent, qui doivent être considérés comme illustratifs et non limitatifs.

### Légendes des Figures

**Figure 1** **:** Effet du naratriptan sur le tonus vasculaire des veines sous-cutanées humaines ex-vivo en fonction du temps.
**Figure 2** **:** Effet de quatre triptans sur le tonus vasculaire de veines sous-cutanées humaines, ex-vivo.
**Figure 3** **:** Effet de quatre triptans sur le tonus vasculaire d'artères sous-cutanées humaines, ex-vivo.

### EXEMPLES

### Exemple 1 : Evaluation de l'effet du naratriptan sur le tonus vasculaire des veines sous-cutanées humaines ex vivo en utilisant la technique de myographie

L'effet du chlorhydrate de naratriptan a été évalué en dose-réponse sur des veines sous-cutanées humaines isolées, par la mesure d'une tension lors de la contraction du vaisseau *ex-vivo* en utilisant la technique de myographie.

### Matériel et méthodes

Les veines humaines sous-cutanées sont isolées à partir de peau saine humaine provenant de déchets opératoires de chirurgie plastique (plastie abdominale). A réception, le prélèvement de peau est maintenu à 4°C dans un récipient contenant une solution physiologique saline (PSS) 1X à pH=7,4. La dissection, l'isolement et le montage des veines se font dans du PSS pH=7,4 froid avec des pinces droites en utilisant un stéréomicroscope. Une fois la veine isolée et nettoyée de la graisse et du tissu conjonctif qui l'entourent, un segment de 2mm de long et d'un diamètre compris entre 100 et 600µm est monté dans la cuve du myographe entre deux fils de Tungstène de 40µm fixés sur des mâchoires amovibles reliées au capteur de tension et est maintenue dans une solution physiologique saline (PSS), à pH=7.4 à 37°C. Pour la réalisation de l'étude de myographie, la veine est placée à une pré-tension (g) de travail correspondant à la pression physiologique de 18mmHg. La pré-tension est fixée par action mécanique pour permettre un écartement des fils de Tungstène (40µm). La viabilité du vaisseau est vérifiée par dépolarisation de la membrane des cellules de muscles lisses du vaisseau en augmentant la concentration en potassium dans la cuve. Pour cela, la solution de PSS contenu dans chaque cuve est remplacée par une solution contenant 80mM de KCl à pH=7.4 et préchauffée à 37°C. Cette étape doit être répétée 2 fois, avec un lavage au PSS 1X entrainant une relaxation entre chaque contraction. A ce stade, s'il n'y a pas de modification de la tension mesurée le vaisseau isolé est considéré comme non viable. Le naratriptan a été évalué en dose réponse (n=1) afin de déterminer son intensité maximale de contraction (Emax) et sa puissance (EC50). Pour cela, le vaisseau a été traité avec des doses croissantes successives de produit ajoutées toutes les 3 minutes dans la cuve de myographie. Les données de tension (g) sont successivement converties en:
- Tension (mN) 1g = 9.81 mN
- Tension appliquée sur la paroi du vaisseau (mN/mm) : en divisant la valeur de tension en mN par 2 fois la longueur du vaisseau, soit 4 mm.

Les données sont ensuite exprimées pour le produit vasoconstricteur en pourcentage (%) de contraction en prenant comme valeur 100%, la valeur obtenue pour le traitement avec le KCl 80mM lors de l'étape 1 de vérification de viabilité du vaisseau.

### Résultats

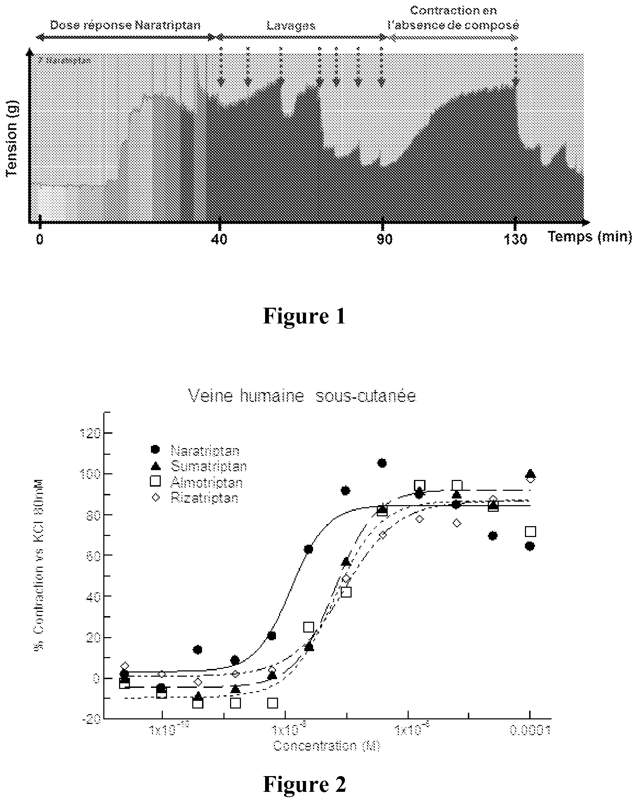

La **figure 1** représente la visualisation des données brutes obtenues lors de l'évaluation du naratriptan en dose-réponse. La tension (g) mesurée par le capteur est représentée en fonction du temps. On visualise la contraction du vaisseau par ajout de doses croissantes du composé toutes les 3 minutes, suivi de lavages successifs en fin d'étude. Ainsi, un premier lavage de la cuve est effectué 3 minutes après l'ajout de la dose maximale de produit dans la cuve de myographie (suppression du produit). A cette étape et en l'absence du composé vasoconstricteur dans la cuve, le vaisseau doit se relaxer spontanément jusqu'à retrouver son tonus vasculaire normal ou physiologique. Comme présentée sur la **figure 1**, la relaxation du vaisseau est très lente voire impossible après une contraction provoquée avec le naratriptan. On constate également qu'après plusieurs lavages successifs, le vaisseau tend à se recontracter jusqu'à atteindre l'intensité maximale de contraction obtenue avec le KCl, alors qu'il n'est plus en présence du composé testé. Le naratriptan montre un effet persistant sur la veine humaine après plusieurs lavages successifs et ce jusqu'à 90 minutes après l'élimination du composé dans la cuve.

Ces résultats montrent que la contraction induite par le naratriptan est persistante dans le temps, ce qui est très avantageux pour le traitement de la rosacée.

### Exemple 2 : Evaluation de l'effet de 4 triptans sur le tonus vasculaire de vaisseaux sanguins humains sous-cutanés (veine et artère) ex-vivo en utilisant la technique de myographie

### Matériel et méthodes

La méthode utilisée dans cette étude est identique à celle utilisée dans l'exemple 1. Pour la réalisation des études de myographie, la veine est placée à une pré-tension (g) correspondant à une pression physiologique de 18mmHg alors que l'artère est placée à une pré-tension de 90mmHg car la paroi d'une artère est plus épaisse que la paroi d'une veine.

L'appareil de myographie dispose de quatre cuves indépendantes. Par conséquent, quatre morceaux de vaisseau ont été montés en parallèle dans chaque étude. Chaque composé a été évalué en dose réponse sur chaque type de vaisseau (n=1). Pour cela, le vaisseau a été traité avec des concentrations croissantes et successives de produit ajoutées toutes les 3 minutes dans la cuve de myographie. Les composés testés sont le naratriptan, le sumatriptan, l'almotriptan et Rizatriptan.

Seuls trois composés (naratriptan, sumatriptan et rizatriptan) ont pu être évalués dans cette étude sur les artères, car la réponse de l'artère montée dans la cuve 2 s'est avérée très faible en intensité lors de la première étape de «contrôle qualité» de traitement au KCl 80mM.

### Résultats

L'intensité de contraction ainsi que la puissance (EC50) de chaque composé sont indiquées dans le tableau ci-dessous :

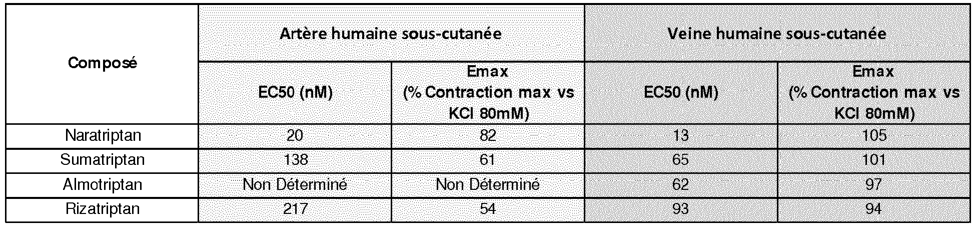

Sur les veines : Les résultats obtenus montrent que les composés induisent une vasoconstriction dose dépendante de la veine humaine sous-cutanée (**Figure 2**).

Le naratriptan présente un fort potentiel vasoconstricteur (EC50 = 13nM) avec une intensité maximale (Emax) de 105% sur la veine sous-cutanée humaine, supérieurs aux valeurs obtenues avec les autres triptans.

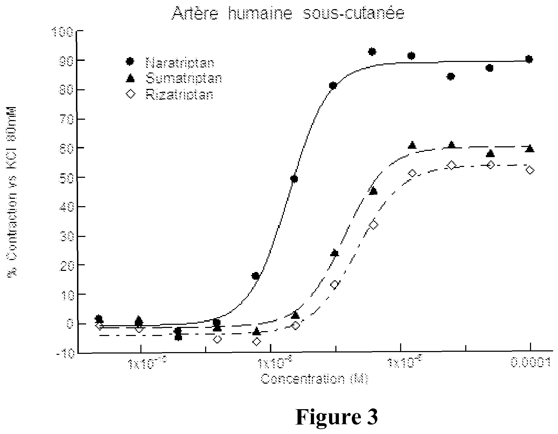

Sur les artères : Les résultats obtenus montrent que les composés induisent également une vasoconstriction dose dépendante de l'artère humaine sous-cutanée (**Figure 3**). Les résultats obtenus avec le sumatriptan (EC50 = 138nM ; Emax=70%) sont en accord avec ceux décrits dans la littérature (Gupta et al., J Hypertens. 2006 Jul; 24(7):1345-53).

L'intensité de contraction maximale sur l'artère est obtenue avec le naratriptan (Emax=82%). Ce dernier est également le vasoconstricteur le plus puissant (EC50=20nM).

Parmi les 4 triptans évalués, le naratriptan présente donc la meilleure activité en termes de puissance et d'efficacité à la fois sur la veine et l'artère sous-cutanée humaine.

### Exemple 3 : Test d'inflammation neurogènique

L'inflammation neurogénique induite après une seule application de reziniferatoxine (RTX) sur l'oreille de souris est inhibée de façon significative par 1% de naratriptan jusqu'à 45 minutes après traitement dans le véhicule acétone. Le composé a ensuite été testé dans le véhicule eau/ethanol afin d'augmenter la solubilité. Le candidat a montré une inhibition significative de l'inflammation neurogénique après application à 1% par voie topique après 15 minutes de traitement.

### Exemple 4 : Efficacité du naratriptan sur des patients atteints de rosacée

Une étude randomisée, en double aveugle et en intra-individuelle chez des 25 sujets présentant une rosacée de type I est réalisée. Cette étude comporte une période de sélection de 4 semaines maximum, une période de traitement de 4 semaines et une période de suivi de 2 à 4 semaines.

Pour répondre à la question de l'exposition systémique et de l'efficacité, deux zones sont traitées avec le naratriptan une fois par jour pendant 4 semaines: un hémi-visage et une surface équivalente à un hémi-visage dans le dos. Cela permet d'être dans les conditions maximisées pour la rosacée en terme de surface d'application. Les deux autres zones symétriques, sur le visage et sur le dos sont traitées avec le véhicule. Les évaluations cliniques (échelle de scores de l'érythème) de l'érythème et des flushs (fréquence et réponse dans un modèle de flush induit) et biophysiques (colorimétrie et photographies) sont conduites au niveau du visage. La pénétration cutanée et les mesures de pharmacodynamie peuvent être réalisées au niveau du dos des sujets par tape-stripping et/ou biopsies. La sécurité générale et la tolérance locale au produit sont évaluées tout au long de l'étude (survenue des évènements indésirables, bilans biologiques, suivi cardiaque, évaluation de l'irritation, etc). Un suivi de 2 à 4 semaines après la fin du traitement est réalisé.

### Exemple 5 : Pénétration comparative du naratriptan et de l'almotriptan dans la peau humaine

L'étude de la libération et de la pénétration sur peau humaine « dermatomée » (Cellules de Franz) a montré que les 2 molécules à 2% dans le PBS sont capables d'atteindre leur cible mais qu'une quantité de naratriptan nettement plus importante comparée à celle de l'almotriptan est retrouvée dans la peau humaine totale.

Tableau résumant les données de l'étude de libération / pénétration :

| | | Chlorhydrate de naratriptan | | Maléate d'almotriptan | |
|---|---|---|---|---|---|
| | | Moyenne | Erreur Standard | Moyenne | Erreur Standard |
| | Quantité appliquée | 184.37 | 1.46 | 151.18 | 1.03 |
| Non absorbé | µg/cm² | 162.40 | 2.89 | 139.88 | 0.86 |
| | % dose appliquée | 88.08 | 1.53 | 92.53 | 0.64 |
| Stratum corneum | µg/cm² | 3.17 | 0.36 | 1.50 | 0.54 |
| | % dose appliquée | 1.72 | 0.20 | 0.99 | 0.40 |
| Epiderme | µg/cm² | 2.95 | 0.83 | 0.97 | 0.37 |
| | % dose appliquée | 1.60 | 0.45 | 0.64 | 0.27 |
| Derme | µg/cm² | 0.53 | 0.15 | 0.34 | 0.11 |
| | % dose appliquée | 0.29 | 0.08 | 0.23 | 0.08 |
| Dose Absorbée | µg/cm² | 0.03 | 0.02 | 0.02 | 0.02 |
| | % dose appliquée | 0.02 | 0.01 | 0.01 | 0.01 |
| Total pénétré | µg/cm² | 6.37 | 1.39 | 2.86 | 1.03 |
| | % dose appliquée | 3.46 | 0.76 | 1.89 | 0.74 |
| Balance masse | % dose appliquée | 91.22 | 2.59 | 94.16 | 1.59 |
| Peau Totale | % dose appliquée | 3.61 | | 1.86 | |

En conclusion, le naratriptan est proposé ici pour son efficacité sur l'érythème ainsi que sur les flushs tout en étant bien toléré. Le naratriptan est utile pour le traitement de la rosacée, et plus particulièrement sur l'érythème, que l'on retrouve dans la rosacée érythémotélangiectasique et papulopustulaire, et plus particulièrement sur la rosacée de type I.

Cet effet peut être persistant plusieurs jours voire plusieurs semaines après l'arrêt de l'application du naratriptan empêchant la réapparition de l'érythème.

## Revendications

1. Composition pharmaceutique topique comprenant du naratriptan ou un sel pharmaceutiquement acceptable de celui-ci, dans un véhicule pharmaceutiquement acceptable, pour une utilisation dans le traitement et/ou la prévention de la rosacée.

2. Composition pour une utilisation dans le traitement et/ou la prévention de la rosacée selon la revendication 1, la rosacée étant une rosacée de type I et II.

3. Composition pour une utilisation dans le traitement et/ou la prévention de la rosacée selon la revendication 2, la rosacée étant une rosacée de type I ou rosacée érythémotélangiectasique.

4. Composition pour une utilisation dans le traitement et/ou la prévention de la rosacée selon l'une des revendications 1 à 3, ladite composition comprenant du chlorhydrate de naratriptan.

5. Composition pour une utilisation dans le traitement et/ou la prévention de la rosacée selon l'une des revendications 1 à 4, la concentration en naratriptan, ou sel de naratriptan, étant comprise entre 0,0001 % et 5% en poids, par rapport au poids total de la composition.

6. Composition pour une utilisation dans le traitement et/ou la prévention de la rosacée selon l'une des revendications 1 à 5, la composition étant sous la forme d'une pommade, une crème, une lotion ou un gel.

## Patentansprüche

1. Topische pharmazeutische Zusammensetzung, umfassend Naratriptan oder ein pharmazeutisch verträgliches Salz davon, in einem pharmazeutisch verträglichen Träger für eine Verwendung in der Behandlung und/oder Prävention der Rosacea.

2. Zusammensetzung für eine Verwendung in der Behandlung und/oder Prävention der Rosacea gemäß Anspruch 1, wobei die Rosacea eine Rosacea Typ I und II ist.

3. Zusammensetzung für eine Verwendung in der Behandlung und/oder Prävention der Rosacea gemäß Anspruch 2, wobei die Rosacea eine Rosacea Typ I oder erythematöse-teleangiektatische Rosacea ist.

4. Zusammensetzung für eine Verwendung in der Behandlung und/oder Prävention der Rosacea gemäß einem der Ansprüche 1 bis 3, wobei besagte Zusammensetzung Naratriptan-Hydrochlorid umfasst.

5. Zusammensetzung für eine Verwendung in der Behandlung und/oder Prävention der Rosacea gemäß einem der Ansprüche 1 bis 4, wobei die Konzentration von Naratriptan oder vom Naratriptansalz zwischen 0,0001 Gew.-% und 5 Gew.-% bezogen auf das Gesamtgewicht der Zusammensetzung beträgt.

6. Zusammensetzung für eine Verwendung in der Behandlung und/oder Prävention der Rosacea gemäß einem der Ansprüche 1 bis 5, wobei die Zusammensetzung in Form einer Salbe, einer Creme, einer Lotion oder eines Gels vorliegt.

## Claims

1. A topical pharmaceutical composition comprising naratriptan or a pharmaceutically acceptable salt thereof, in a pharmaceutically acceptable carrier, for use in the treatment and/or prevention of rosacea.

2. The composition for use in the treatment and/or prevention of rosacea according to claim 1, the rosacea being Type I or II rosacea.

3. The composition for use in the treatment and/or prevention of rosacea according to claim 2, the rosacea being Type I rosacea or erythematotelangiectatic rosacea.

4. The composition for use in the treatment and/or prevention of rosacea according to one of claims 1 to 3, said composition comprising naratriptan hydrochloride.

5. The composition for use in the treatment and/or prevention of rosacea according to one of claims 1 to 4, the concentration of naratriptan or salt of naratriptan being comprised between 0.0001 % and 5% by weight relative to the total weight of the composition.

6. The composition for use in the treatment and/or prevention of rosacea according to one of claims 1 to 5, the composition being in the form of an ointment, cream, lotion or gel.
